# EUROPÄISCHE PATENTSCHRIFT

(11) **EP 1 021 420 B1**
(45) Veröffentlichungstag und Bekanntmachung des Hinweises auf die Patenterteilung: **26.03.2003**
(21) Anmeldenummer: 98958843.9
(22) Anmeldetag: 25.09.1998
(51) Int. Cl.: C07D 249/12

(54) **VERFAHREN ZUR HERSTELLUNG VON TRIAZOLINTHION-DERIVATEN**
METHOD FOR PRODUCING TRIAZOLINETHIONE DERIVATIVES
PROCEDE DE PRODUCTION DE DERIVES DE TRIAZOLINETHIONE

(30) Priorität: 08.10.1997 DE 19744401
(43) Veröffentlichungstag der Anmeldung: 26.07.2000
(73) Patentinhaber: Bayer CropScience AG, 40789 Monheim (DE)
(72) Erfinder: LANTZSCH, Reinhard, D-42115 Wuppertal (DE); JAUTELAT, Manfred, D-51399 Burscheid (DE); HUPPERTS, Achim, D-40217 Düsseldorf (DE); ERDMAN, David, D-51061 Köln (DE)
(86) Internationale Anmeldenummer: EP9806111
(87) Internationale Veröffentlichungsnummer: WO99018086

(56) Entgegenhaltungen:
- EP-A- 0 784 053
- WO-A-96/16048
- ISAMU ARAI: "Reactions of phenylhydrazinium thiocyanate with ketones and aldehydes" BULLETIN OF THE CHEMICAL SOCIETY OF JAPAN., Bd. 46, Nr. 7, 1973, Seiten 2215-2218, XP002090972 TOKYO JP in der Anmeldung erwähnt
- CHEMICAL ABSTRACTS, vol. 95, no. 23, 7. Dezember 1981 Columbus, Ohio, US; abstract no. 203899, GADZHIEV G. ET AL.: "Reaction of ethanol hydrazones with isocyanates" XP002090973 & ZH. ORG. KHIM., Bd. 17, Nr. 8, 1981, Seiten 1784-1785, USSR
- CHEMICAL ABSTRACTS, vol. 88, no. 11, 13. März 1978 Columbus, Ohio, US; abstract no. 74395, WAKABAYASHI O. ET AL.: "1,2-Substituted-3-alkoxycarboimino-4-subs tituted s-triazolidine-5-thiones" XP002090974 & JP 52 113972 A (MITSUBISHI CHEMICAL INDUSTRIES CO., LTD.)

## Beschreibung

Die vorliegende Erfindung betrifft ein neues Verfahren zur Herstellung von Triazolinthion-Derivaten, die als Wirkstoffe mit mikrobiziden, insbesondere fungiziden Eigenschaften bekannt sind.

Es ist bereits bekannt, daß sich Triazolinthion-Derivate herstellen lassen, indem man die entsprechenden Triazol-Derivate entweder nacheinander mit starken Basen und Schwefel umsetzt und dann hydrolysiert oder direkt mit Schwefel bei hohen Temperaturen umsetzt und dann mit Wasser behandelt (vergl. WO-A 96-16 048). Nachteilig an diesen Verfahren ist aber. daß die gewünschten Produkte nur in relativ niedrigen Ausbeuten anfallen, oder daß Umsetzungsbedingungen erforderlich sind, die im technischen Maßstab nur schwierig einzuhalten sind.

Weiterhin wurde schon beschrieben, daß sich bestimmte, in 3-Position substituierte 1,2,4-Triazolin-5-thione dadurch herstellen lassen, daß man N-Chlorthioformyl-N-(1-chloralk-1-en)-amine mit Carbonylhydrazin-Derivaten umsetzt (vergl. DE-A 197 01 032, DE-A 196 01 189 und EP-A 0 784 053). Die Synthese von entsprechenden Substanzen, die keinen Substituenten in der 3-Position enthalten, wird jedoch nicht erwähnt.

Ferner geht aus Bull. Chem. Soc. Japan 46, 2215 (1973) hervor, daß sich in 3-Position substituierte Triazolinthione synthetisieren lassen, indem man Phenylhydrazin mit Natriumthiocyanat und Ketonen oder Aldehyden in Gegenwart von Salzsäure umsetzt und die dabei anfallenden, in 3-Position substituierten Triazolidinthione mit oxidierenden Reagenzien behandelt. Beeinträchtigend an diesem Verfahren ist, daß sehr lange Reaktionszeiten erforderlich sind und keine in 3-Stellung unsubstituierten Triazolinthione auf diese Weise zugänglich sind.

Schließlich ist auch schon bekannt, daß man 2-(1-Chlor-cyclopropyl)-1-(2-chlorphenyl)-3-(1,2,4-triazol-1-yl)-propan-2-ol erhält, wenn man [1-(2-Chlor-phenyl)-2-(1-chlor-cyclopropyl)-2-hydroxy]-propyl-1-hydrazin mit Formamidinacetat umsetzt (vergl. DE-A 40 30 039). Thiono-Derivate von Triazolen sind nach dieser Methode allerdings nicht zugänglich.

Es wurde nun gefunden, daß sich Triazolinthion-Derivate der Formel 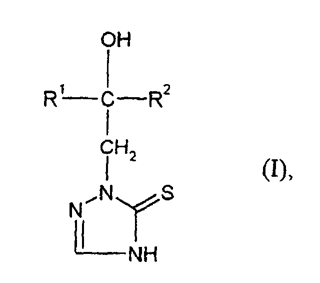 in welcher
- R¹: für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffätomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl
oder
fiir Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
- R²: für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
fiir geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
fiir Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
herstellen lassen, indem man
a) in einer ersten Stufe Hydrazin-Derivate der Formel 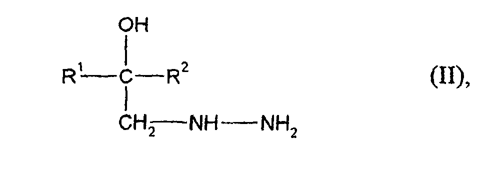 in welcher
   R¹ und R² die oben angegebenen Bedeutungen haben, mit Carbonylverbindungen der Formel 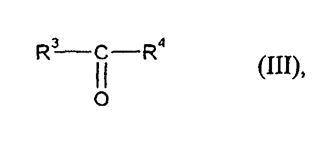 in welcher
   - R³: für Methyl, Ethyl, n-Propyl, Isopropyl, n-Butyl, tert.-Butyl oder Phenyl steht und
   - R⁴: für Wasserstoff, Methyl, Erthyl oder n-Propyl steht oder
   R³ und R⁴ gemeinsam für eine -(CH₂)₅-Kette stehen,
   und mit Thiocyanat der Formel

   X-SCN (IV),

   in welcher
   - X: für Natrium, Kalium oder Ammonium steht,
   gegebenenfalls in Gegenwart eines Verdünnungsmittels und gegebenenfalls in Gegenwart einer Säure umsetzt und
b) die entstandenen Triazolidinthion-Derivate der Formel 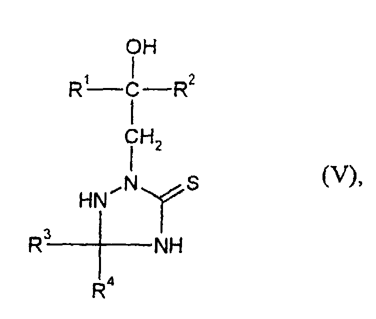 in welcher
   R¹, R², R³ und R⁴ die oben angegebenen Bedeutungen haben,
   mit Ameisensäure, gegebenenfalls in Gegenwart eines Katalysators und gegebenenfalls in Gegenwart eines Verdünnungsmittels umsetzt.

Es ist als ausgesprochen überraschend zu bezeichnen, daß sich Triazolinthion-Derivate der Formel (I) nach dem erfindungsgemäßen Verfahren in wesentlich höheren Ausbeuten oder unter erheblich einfacheren Bedingungen herstellen lassen als nach den bisher bekannten Methoden. Unerwartet ist auch, daß bei der Durchführung der zweiten Stufe des erfindungsgemäßen Verfahrens ein Austausch der 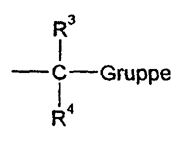 gegen eine Methylen-Gruppe mit hoher Selektivität erfolgt.

Das erfindungsgemäße Verfahren zeichnet sich durch eine Reihe von Vorteilen aus. So ermöglicht es, wie schon erwähnt, die Synthese von Triazolinthionen der Formel (I) in hoher Ausbeute. Günstig ist auch, daß die benötigten Ausgangsstoffe und Reaktionskomponenten einfach herstellbar und auch in größeren Mengen verfügbar sind. Ein weiterer Vorteil besteht schließlich darin, daß die Durchführung der einzelnen Reaktionsschritte und die Isolierung der Reaktionsprodukte keinerlei Schwierigkeiten bereitet.

Verwendet man 2-(1-Chlor-cyclopropyl)-3-(2-chlor-phenyl)-2-hydroxy-propyl-1-hydrazin als Ausgangssubstanz und setzt diese in der ersten Stufe mit Aceton und Kaliumthiocyanat um und läßt das dabei anfallende Triazolidinthion-Derivat in der zweiten Stufe mit Ameisensäure reagieren, so kann der Verlauf des erfindungsgemäßen Verfahrens durch das folgende Formelschema veranschaulicht werden.

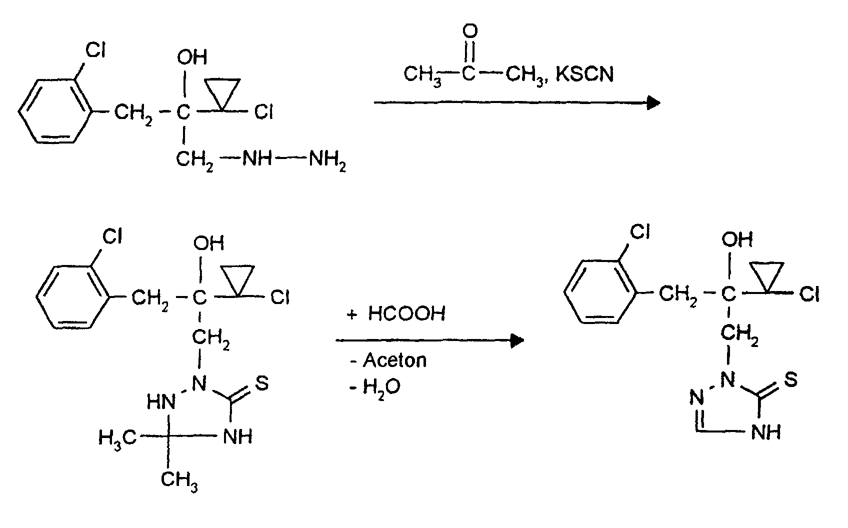

Die bei der Durchführung des erfindungsgemäßen Verfahrens als Ausgangsstoffe benötigten Hydrazin-Derivate sind durch die Formel (II) allgemein definiert. Bevorzugt einsetzbar sind Verbindungen der Formel (II), in denen
- R¹: für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-Cyclopropyl, Cyclopropyl, 1-Methylcyclopentyl oder 1-Ethyl-cyclopentyl steht, oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
fiir Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
- R²: für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-cyclopropyl, Cyclopropyl, 1-Methylcyclopentyl oder 1-Ethyl-cyclopentyl steht, oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano.

Die Hydrazin-Derivate der Formel (II) sind bekannt oder lassen sich nach prinzipiell bekannten Verfahren herstellen (vergl. DE-A-40 30 039).

So erhält man Hydrazin-Derivate der Formel (II), indem man 1-Chlor-2-hydroxy-ethan-Derivate der Formel 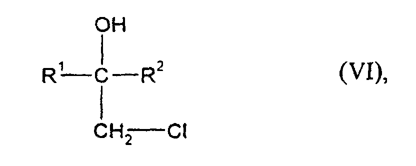 oder Oxiran-Derivate der Formel 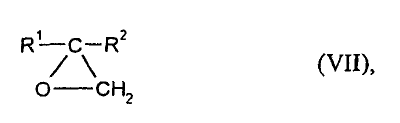 in welchen
R¹ und R² jeweils die oben angegebenen Bedeutungen haben,
mit Hydrazinhydrat gegebenenfalls in Gegenwart eines Verdünnungsmittels umsetzt.

Die 1-Chlor-2-hydroxy-ethan-Derivate der Formel (VI) und auch die Oxiran-Derivate der Formel (VII) sind bekannt oder lassen sich nach prinzipiell bekannten Verfahren herstellen (vergl. DE-A-40 30 039 und EP-A 0 2397 345).

Als Verdünnungsmittel kommen bei dem obigen Verfahren zur Herstellung von Hydrazin-Derivaten der Formel (II) alle üblichen inerten, organischen Solventien in Frage. Vorzugsweise verwendbar sind Alkohole, wie Methanol, Ethanol oder n-Butanol, ferner Ether, wie Dioxan oder Methyl-tert.-butylether, und weiterhin aromatische Kohlenwasserstoffe, wie Benzol, Toluol oder Xylol. Es ist aber auch möglich, ohne zusätzliches Lösungsmittel zu arbeiten. In diesem Fall setzt man Hydrazinhydrat im Überschuß ein, so daß es sowohl als Reaktionskomponente als auch als Verdünnungsmittel fungiert.

Die Reaktionstemperaturen können bei der Herstellung von Hydrazin-Derivaten der Formel (II) nach dem obigen Verfahren in einem bestimmten Bereich variiert werden. Im allgemeinen arbeitet man bei Temperaturen zwischen 60°C und 120°C, vorzugsweise zwischen 70°C und 110°C.

Bei der Herstellung von Hydrazin-Derivaten der Formel (II) nach dem obigen Verfahren setzt man auf 1 mol an 1-Chlor-2-hydroxy-ethan-Derivat der Formel (VI) oder an Oxiran-Derivat der Formel (VII) im allgemeinen 1 bis 20 mol, vorzugsweise 5 bis 15 mol an Hydrazinhydrat ein. Die Aufarbeitung erfolgt nach üblichen Methoden. Im allgemeinen verfährt man in der Weise, daß man das Reaktionsgemisch mit einem mit Wasser wenig mischbaren organischen Solvens, wie Methyl-tert.-butylether oder Toluol versetzt, die wäßrige Phase abtrennt, die organische Phase wäscht und trocknet.

Die bei der Durchführung der ersten Stufe des erfindungsgemäßen Verfahrens als Reaktionskomponenten benötigten Carbonylverbindungen sind durch die Formel (III) allgemein definiert. Bevorzugt einsetzbar sind Carbonylverbindungen der Formel (III), in welcher
- R³: für Methyl. Ethyl, n-Propyl, Isopropyl, n-Butyl, tert.-Butyl oder Phenyl steht und
- R⁴: für Wasserstoff, Methyl, Ethyl oder n-Propyl steht oder
- R³ und R⁴: gemeinsam für eine -(CH₂)₅-Kette stehen.

Als Beispiele für Carbonylverbindungen der Formel (III) seien genannt:
Acetaldehyd
Propionaldehyd
Benzaldehyd
Aceton
Diethylketon
Methyl-ethyl-keton
Di-n-propyl-keton
Pinakolin
Acetophenon und
Cyclohexanon

Die Carbonylverbindungen der Formel (III) und auch die Thiocyanate der Formel (IV) sind bekannt.

Als Verdünnungsmittel kommen bei der Durchführung der ersten Stufe des erfindungsgemäßen Verfahrens alle für derartige Umsetzungen üblichen, inerten organischen Solventien in Betracht. Vorzugsweise verwendbar sind aromatische Kohlenwasserstoffe, wie Benzol, Toluol oder Xylol, ferner Ether, wie Dioxan, Methyl-tert.butylether, 1,2-Dimethoxy-ethan oder Methyl-tert.-amylether, weiterhin Ester, wie Essigsäureethylester oder Essigsäurebutylester, und außerdem Alkohole, wie Propanol, Butanol oder Pentanol. Es kann aber auch im Überschuß eingesetzte Carbonylverbindung der Formel (III) als Verdünnungsmittel fungieren.

Als Säuren kommen bei der Durchführung der ersten Stufe des erfindungsgemäßen Verfahrens übliche anorganische oder organische Säuren in Frage. Vorzugsweise verwendbar sind Salzsäure, Schwefelsäure oder p-Toluolsulfonsäure.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 0°C und 120°C, vorzugsweise zwischen 10°C und 110°C.

Sowohl bei der Durchführung der ersten als auch der zweiten Stufe des erfindungsgemäßen Verfahrens arbeitet man im allgemeinen unter Atmosphärendruck. Es ist aber auch möglich, unter erhöhtem Druck oder, sofern keine gasförmigen Komponenten an der Reaktion beteiligt sind, auch unter vermindertem Druck zu arbeiten.

Bei der Durchführung der ersten Stufe des erfindungsgemäßen Verfahrens setzt man auf 1 mol an Hydrazin-Derivat der Formel (II) im allgemeinen 1 bis 2 mol an Carbonylverbindung der Formel (III) und 1 bis 2 mol an Thiocyanat der Formel (IV) ein. Die Aufarbeitung erfolgt nach üblichen Methoden. Im allgemeinen geht man so vor, daß man das Reaktionsgemisch mit Wasser wäscht, die organische Phase trocknet und einengt und den verbleibenden Rückstand nach üblichen Methoden, zum Beispiel durch Umkristallisation, von unerwünschten Bestandteilen befreit.

Die bei der Durchführung der zweiten Stufe des erfindungsgemäßen Verfahrens als Ausgangsstoffe benötigten Triazolidinthion-Derivate sind durch die Formel (V) allgemein definiert. In dieser Formel haben R¹, R², R³ und R⁴ vorzugsweise diejenigen Bedeutungen, die bereits im Zusammenhang mit der Beschreibung der Hydrazin-Derivate der Formel (II) bzw. der Carbonylverbindungen der Formel (III) vorzugsweise für diese Reste genannt wurden.

Die Triazolidinthion-Derivate der Formel (V) sind bisher nocht nicht bekannt. Sie lassen sich durch die Umsetzung in der ersten Stufe des erfindungsgemäßen Verfahrens herstellen.

Als Katalysatoren kommen bei der Durchführung der zweiten Stufe des erfindungsgemäßen Verfahrens alle für derartige Umsetzungen üblichen Reaktionsbeschleuniger in Frage. Vorzugsweise verwendbar sind Säuren, wie Salzsäure, Schwefelsäure oder p-Toluolsulfonsäure, und ferner Metalloxide, wie amorphes Titandioxid.

Als Verdünnungsmittel kommen bei der Durchführung der zweiten Stufe des erfindungsgemäßen Verfahrens alle für derartige Umsetzungen üblichen, schwach polaren organischen Solventien in Betracht. Vorzugsweise verwendbar sind Alkohole, wie Propanol, Butanol oder Pentanol, weiterhin Ester, wie Essigsäureethylester, Essigsäurebutylester oder Ameisensäure-isobutylester, ferner Ether, wie 1,2-Dimethoxy-ethan, Methyl-tert.-butylether oder Methyl-tert.-amylether, und auch Ameisensäure im Überschuß.

Die Reaktionstemperaturen können bei der Durchführung der zweiten Stufe des erfindungsgemäßen Verfahrens innerhalb eines bestimmten Bereiches variiert werden. Im allgemeinen arbeitet man bei Temperaturen zwischen 80°C und 150°C, vorzugsweise zwischen 90°C und 130°C.

Bei der Durchführung der zweiten Stufe des erfindungsgemäßen Verfahrens setzt man auf 1 mol an Triazolidinthion-Derivat der Formel (V) einen Überschuß, im allgemeinen 5 bis 50 mol an Ameisensäure sowie gegebenenfalls eine geringe Menge an Katalysator ein. Die Aufarbeitung erfolgt nach üblichen Methoden. Im allgemeinen verfährt man in der Weise, daß man das Reaktionsgemisch gegebenenfalls nach vorherigem Verdünnen mit einem mit Wasser wenig mischbaren organischen Solvens, mit wäßriger Salzlösung ausschüttelt, die organische Phase trocknet und einengt. Gegebenenfalls dann noch vorhandene Verunreinigungen lassen sich nach üblichen Methoden, wie Umkristallisation, abtrennen.

In einer besonderen Variante läßt sich das erfindungsgemäße Verfahren in der Weise durchführen, daß man 1-Chlor-2-hydroxy-ethan-Derivate der Formel (VI) oder Oxiran-Derivate der Formel (VII) mit Hydrazinhydrat umsetzt und die dabei entstehenden Hydrazin-Derivate der Formel (II) dann ohne vorherige Isolierung und Reinigung weiter umsetzt. Demgemäß lassen sich Triazolinthion-Derivate der Formel (I) auch dadurch herstellen, daß man
- 1-Chlor-2-hydroxy-ethan-Derivate der Formel 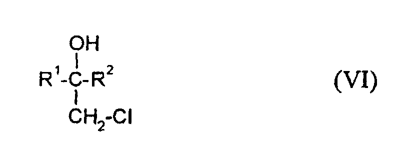 oder Oxiran-Derivate der Formel 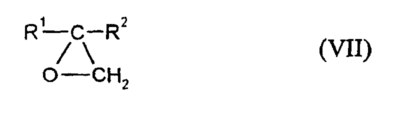 in welchen
   R¹ und R² jeweils die oben angegebenen Bedeutungen haben,
   mit Hydrazinhydrat gegebenenfalls in Gegenwart eines Verdünnungsmittels umsetzt und die dabei entstehenden
- Hydrazin-Derivate der Formel 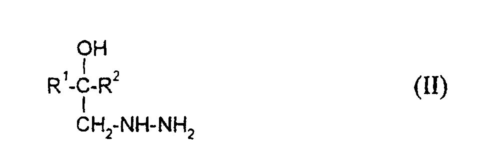 in welcher
   R¹ und R² die oben angegebenen Bedeutungen haben,
   ohne vorherige Isolierung mit Carbonylverbindungen der Formel 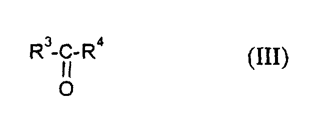 in welcher R³ und R⁴ die oben angegebenen Bedeutungen haben,
   und mit Thiocyanat der Formel

   X-SCN (IV)

   in welcher
   - X: für Natrium, Kalium oder Ammonium steht,
   gegebenenfalls in Gegenwart eines Verdünnungsmittels und gegebenenfalls in Gegenwart einer Säure umsetzt und
- die entstandenen Triazolidinthion-Derivate der Formel 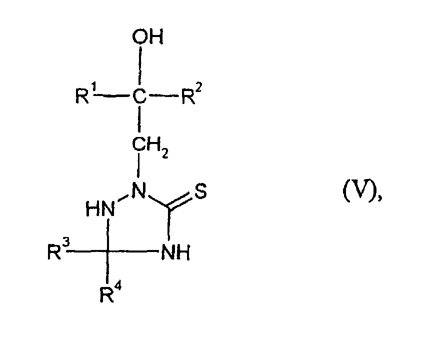 in welcher
   R¹, R², R³ und R⁴ die oben angegebenen Bedeutungen haben,
   mit Ameisensäure, gegebenenfalls in Gegenwart eines Katalysators und gegebenenfalls in Gegenwart eines Verdünnungsmittels umsetzt.

Bei der Durchführung der einzelnen Stufen dieses Verfahrens geht man in der schon oben beschriebenen Weise vor.

Die erfindungsgemäß herstellbaren Triazolinthion-Derivate können in der "Thiono"-Form der Formel 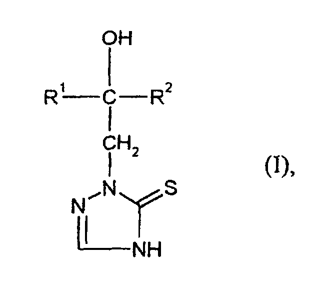 oder in der tautomeren "Mercapto"-Form der Formel

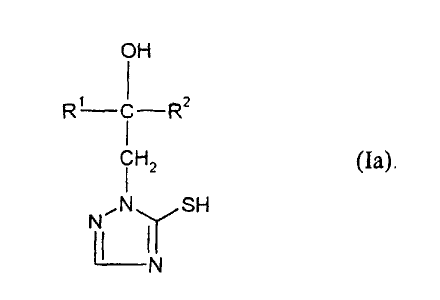

Der Einfachheit halber wird jeweils nur die "Thiono"-Form aufgeführt.

Die erfindungsgemäß herstellbaren Triazolinthion-Derivate sind als Wirkstoffe mit mikrobiziden, insbesondere fungiziden Eigenschaften bekannt (vgl. WO-A 96-16 048).

Die Durchführung des erfindungsgemäßen Verfahrens wird durch die folgenden Beispiele veranschaulicht.

### Herstellungsbeispiele

### Beispiel 1

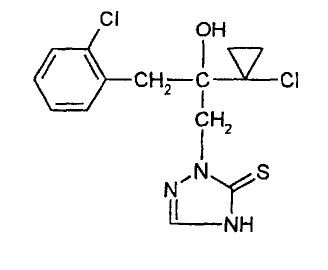

a) Herstellung der Verbindung der Formel 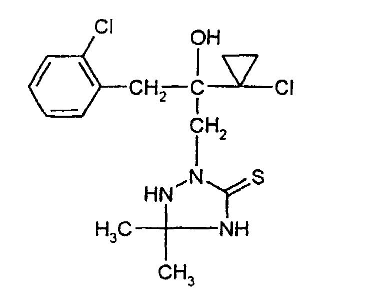
   Ein Gemisch aus 27,5 g (0,1 mol) 2-(1-Chlor-cyclopropyl)-3-(2-chlor-phenyl)-2-hydroxy-propyl-1-hydrazin und 300 ml 2n wäßriger Salzsäure wird unter rühren bei Raumtemperatur zunächst mit 8,2 g (0,14 mol) Aceton und dann mit 13,6 g (0,14 mol) Kaliumthiocyanat versetzt. Danach wird das Reaktionsgemisch mit 100 ml Toluol versetzt und noch 10 Stunden bei Raumtemperatur gerührt. Anschließend wird der entstandene Feststoff abfiltriert, mit Wasser und dann mit Toluol nachgewaschen und getrocknet. Man erhält auf diese Weise 30,7 g (82,1 % der Theorie) an dem Triazolidinthion-Derivat in Form einer farblosen, kristallinen Festsubstanz vom Schmelzpunkt 186 bis 189°C.
b) Herstellung der Verbindung der Formel 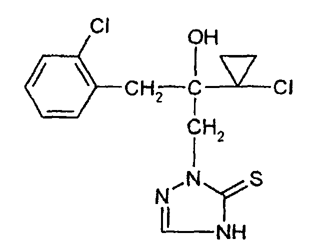
   Ein Gemisch aus 92 g Ameisensäure und 100 ml Ameisensäure-isobutylester wird unter Rühren bei Raumtemperatur mit 18,7 g (0,05 mol) des unter (a) beschriebenen Triazolidinthion-Derivates versetzt. Nach beendeter Zugabe wird das Reaktionsgemisch 17 Stunden unter Rückfluß erhitzt, dann durch Abdestillieren von überschüssiger Ameisensäure und von Lösungsmittel eingeengt und schließlich im Hochvakuum von leicht flüchtigen Bestandteilen befreit. Das verbleibende Produkt wird in Toluol gelöst, die entstehende Lösung wird mit Wasser gewaschen, und die organische Phase wird über Natriumsulfat getrocknet und durch Abdestillieren des Lösungsmittels eingeengt. Man erhält auf diese Weise 14,2 g (76 % der Theorie) an 2-(1-Chlorcyclopropyl)-1-(2-chlor-phenyl)-3-(4,5-dihydro-1,2,4-triazol-5-thiono-1-yl)-propyl-2-ol in Form einer Festsubstanz mit einem Schmelzpunkt von 138 bis 139°C.

### Beispiel 2

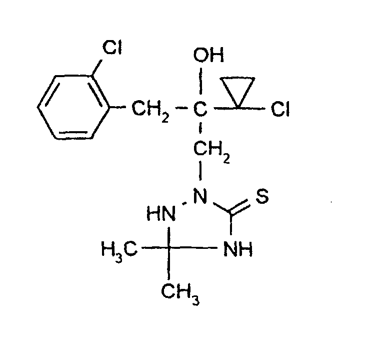

In 25 g (0,5 mol) Hydrazinhydrat werden bei Raumtemperatur und unter Rühren 22 g (= 0,05 mol) eines Produktes, das zu 63,7 % aus 1-Chlor-2-hydroxy-ethan besteht, innerhalb von 15 Minuten eingetropft. Nach beendeter Zugabe wird das Reaktionsgemisch 2 Stunden bei 100°C nachgerührt und dann auf Raumtemperatur abgekühlt. Man verrührt das anfallende Gemisch mit 100 ml Wasser und dekantiert die wäßrige Phase vom festen Niederschlag ab. Dieser Vorgang wird noch einmal mit 100 ml Wasser wiederholt. Das dabei erhaltene Produkt wird mit 150 ml 2n wäßriger Salzsäure, 4,1 g (0,07 mol) Aceton, 6,8 g (0,07 mol) Kaliumthiocyanat und 50 ml Toluol versetzt. Das Reaktionsgemisch wird 12 Stunden bei Raumtemperatur gerührt und dann filtriert. Der Filterrückstand wird nacheinander mit Wasser und Toluol gewaschen und dann getrocknet. Man erhält auf diese Weise 11,6 g an Triazolidinthion-Derivat der oben angegebenen Formel in Form einer farblosen, kristallinen Festsubstanz vom Schmelzpunkt 186 bis 187°C. Die Ausbeute errechnet sich zu 62,0 % der Theorie, bezogen auf eingesetztes 1-Chlor-2-hydroxy-ethan.

Nach der im Beispiel 2 angegebenen Methode werden auch die in der folgenden Tabelle aufgeführten Triazolidinthion-Derivate hergestellt.

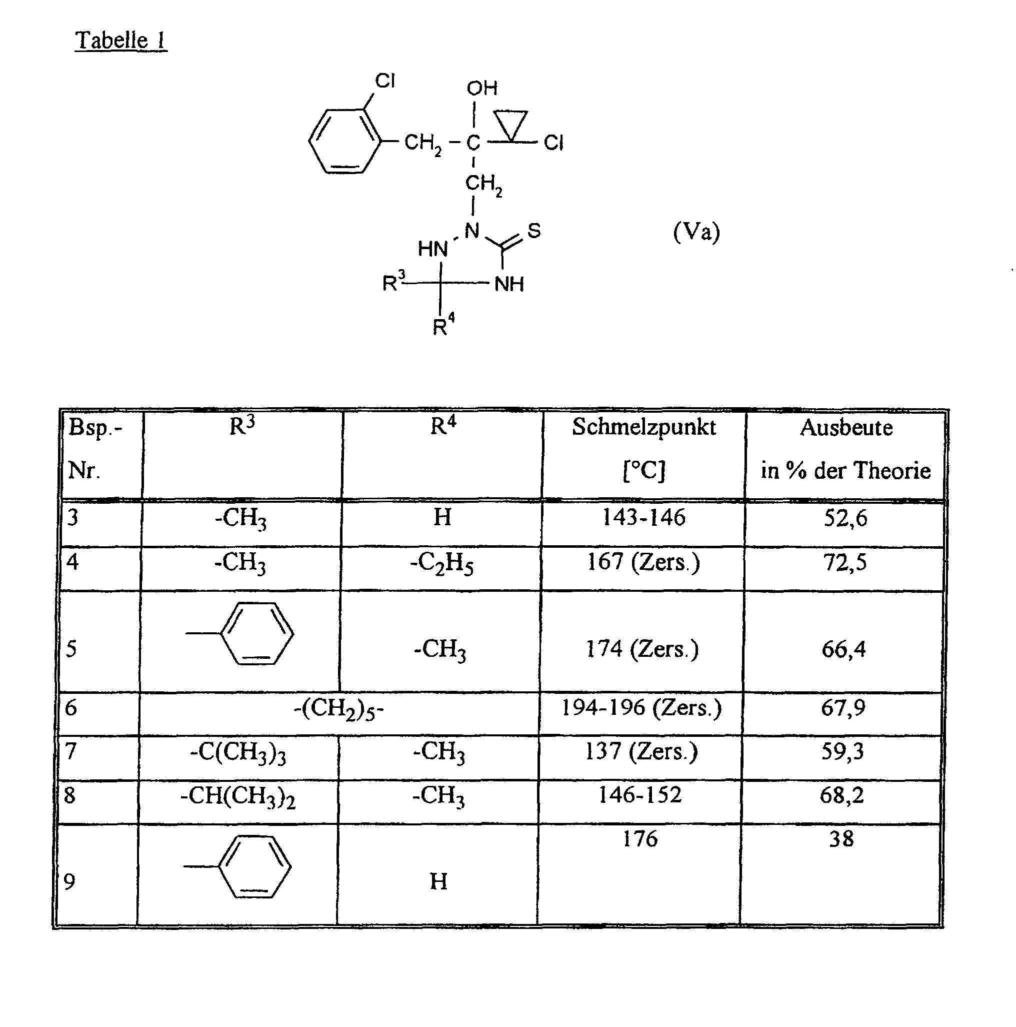

### Vergleichsbeispiele

### Beispiel A

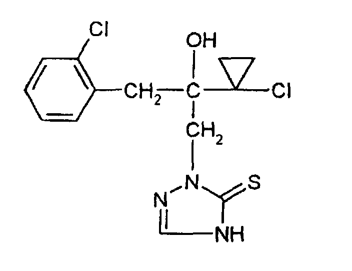

Ein Gemisch aus 3,12 g (10 mmol) 2-(1-Chlor-cyclopropyl)-1-(2-chlorphenyl)-3-(1,2,4-triazol-1-yl)-propan-2-ol und 45 ml absolutem Tetrahydrofuran wird bei -20°C mit 8,4 ml (21 mmol) n-Butyl-lithium in Hexan versetzt und 30 Minuten bei 0°C gerührt. Danach wird das Reaktionsgemisch auf -70°C abgekühlt, mit 0,32 g (10 mmol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g eines Produktes, das gemäß gaschromatografischer Analyse zu 95% aus 2-(1-Chlor-cyclopropyl)-1-(2-chlor-phenyl)-3-(4,5-dihydro-1,2,4-triazol-5-thiono-1-yl)-propan-2-ol besteht. Nach Umkristallisation aus Toluol erhält man diese Substanz als Feststoff, der bei 138 bis 139°C schmilzt.

### Beispiel B

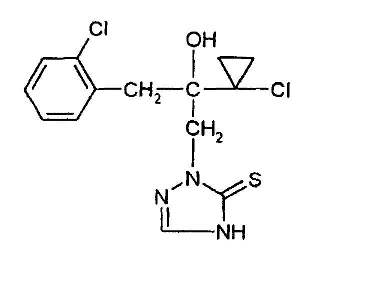

Ein Gemisch aus 3,12 g (10 mmol) 2-(1-Chlor-cyclopropyl)-1-(2-chlor-phenyl)-3-(1,2,4-triazol-1-yl)-propan-2-ol, 0,95 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlor-cyclopropyl)-1-(2-chlorphenyl)-3-(4,5-dihydro-1,2,4-triazol-5-thiono-1-yl)-propan-2-ol in Form einer Festsubstanz, die bei 138-139°C schmilzt.

## Patentansprüche

1. Verfahren zur Herstellung von Triazolinthion-Derivaten der Formel 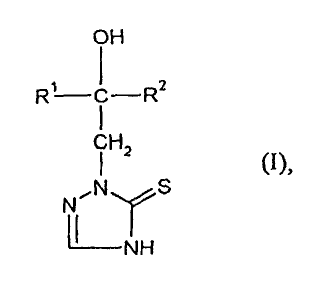 in welcher
R¹ für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
R² für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
**dadurch gekennzeichnet, daß** man
a) in einer ersten Stufe Hydrazin-Derivate der Formel 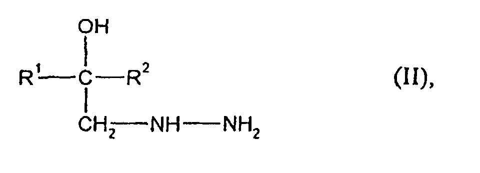 in welcher
R¹ und R² die oben angegebenen Bedeutungen haben, mit Carbonylverbindungen der Formel 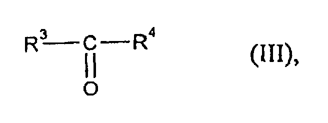 in welcher
R³ für Methyl, Ethyl, n-Propyl, Isopropyl, tert.-Butyl oder Phenyl steht und
R⁴ für Wasserstoff, Methyl, Ethyl oder n-Propyl steht oder
R³ und R⁴ gemeinsam für eine -(CH₂)₅-Kette stehen,
und mit Thiocyanat der Formel
X-SCN (IV),
in welcher
X für Natrium, Kalium oder Ammonium steht,
gegebenenfalls in Gegenwart eines Verdünnungsmittels und gegebenenfalls in Gegenwart einer Säure umsetzt und
b) die entstandenen Triazolidinthion-Derivate der Formel 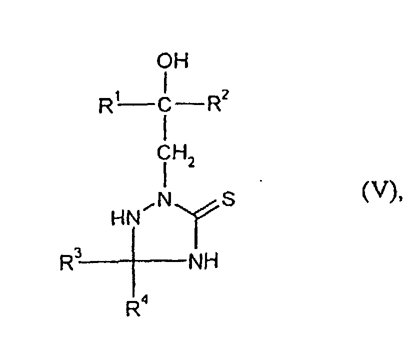
in welcher
R¹, R², R³ und R⁴ die oben angegebenen Bedeutungen haben,
mit Ameisensäure, gegebenenfalls in Gegenwart eines Katalysators und gegebenenfalls in Gegenwart eines Verdünnungsmittels umsetzt.

2. Verfahren gemäß Anspruch 1, **dadurch gekennzeichnet, daß** man 2-(1-Chlor-cyclopropyl)-3-(2-chlor-phenyl)-2-hydroxy-propyl-1-hydrazin der Formel 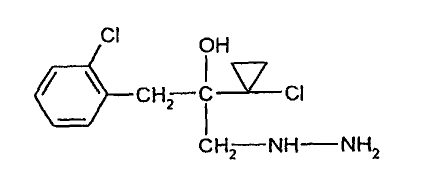 als Ausgangssubstanz einsetzt.

3. Verfahren gemäß Anspruch 1, **dadurch gekennzeichnet, daß** man bei der Durchführung der ersten Stufe Natrium- oder Kaliumthiocyanat als Reaktionskomponente einsetzt.

4. Verfahren gemäß Anspruch 1, **dadurch gekennzeichnet, daß** man bei der Durchführung der ersten Stufe bei Temperaturen zwischen 0°C und 120°C arbeitet.

5. Verfahren gemäß Anspruch 1, **dadurch gekennzeichnet, daß** man bei der Durchführung der zweiten Stufe des erfindungsgemäßen Verfahrens bei Temperaturen zwischen 80°C und 150°C arbeitet.

6. Triazolidinthion-Derivate der Formel 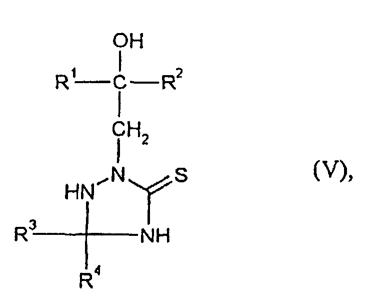 in welcher
R¹, R², R³ und R⁴ die im Anspruch 1 angegebenen Bedeutungen haben.

7. Triazolidinthion-Derivat gemäß Anspruch 6, **gekennzeichnet durch** die Formel

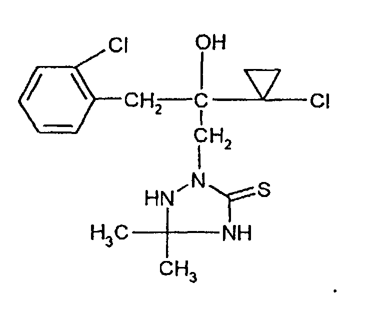

8. Verfahren zur Herstellung von Triazolinthion-Derivaten der Formel 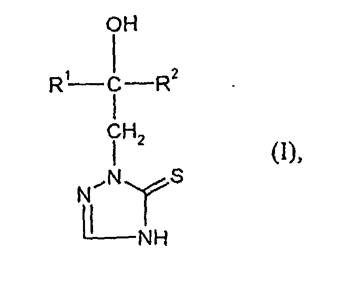 in welcher
R¹ und R² die im Anspruch 1 angegebenen Bedeutungen haben,
**dadurch gekennzeichnet, daß** man
- 1-Chlor-2-hydroxy-ethan-Derivate der Formel 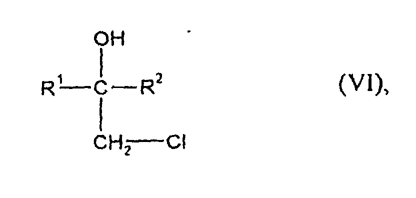 oder Oxiran-Derivate der Formel 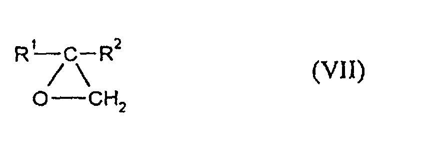 in welcher
R¹ und R² die oben angegebenen Bedeutungen haben,
mit Hydrazinhydrat gegebenenfalls in Gegenwart eines Verdünnungsmittels umsetzt und die dabei entstehenden
- Hydrazin-Derivate der Formel 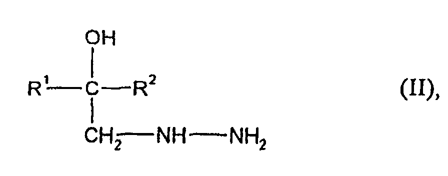 in welcher
R¹ und R² die oben angegebenen Bedeutungen haben,
ohne vorherige Isolierung mit Carbonylverbindungen der Formel 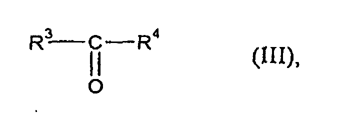 in welcher
R³ und R⁴ die im Anspruch 1 angegebenen Bedeutungen haben,
und mit Thiocyanat der Formel
X-SCN (IV),
in welcher
X für Natrium, Kalium oder Ammonium steht,
gegebenenfalls in Gegenwart eines Verdünnungsmittels und gegebenenfalls in Gegenwart einer Säure umsetzt und
- die entstandenen Triazolidinthion-Derivate der Formel 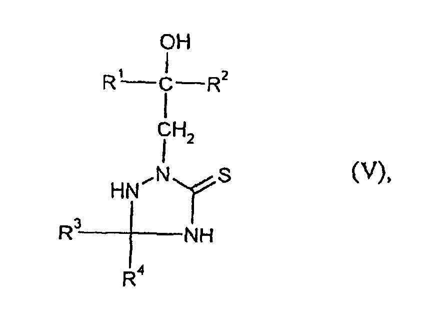 in welcher
R¹, R², R³ und R⁴ die oben angegebenen Bedeutungen haben,
mit Ameisensäure, gegebenenfalls in Gegenwart eines Katalysators und gegebenenfalls in Gegenwart eines Verdünnungsmittels umsetzt.

## Claims

1. Process for preparing triazolinethione derivatives of the formula 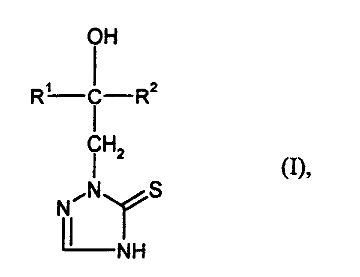 in which
R¹ represents straight-chain or branched alkyl having 1 to 4 carbon atoms, where these radicals may be mono- to tetrasubstituted by identical or different substituents from the group consisting of fluorine, chlorine, bromine, methoxy, ethoxy, propoxy, isopropoxy, alkoximino having 1 or 2 carbon atoms in the alkoxy moiety, cyclopropyl, cyclobutyl, cyclopentyl and cyclohexyl,
or
represents straight-chain or branched alkenyl having 2 to 5 carbon atoms, where each of these radicals may be mono- to trisubstituted by identical or different substituents from the group consisting of fluorine, chlorine, bromine, methoxy, ethoxy, propoxy, isopropoxy, cyclopropyl, cyclobutyl, cyclopentyl and cyclohexyl,
or
represents cycloalkyl having 3 to 6 carbon atoms, where each of these radicals may be mono- to trisubstituted by identical or different substituents from the group consisting of fluorine, chlorine, bromine, cyano, methyl, ethyl, propyl, isopropyl and tert-butyl,
or
represents phenylalkyl having 1 to 4 carbon atoms in the straight-chain or branched alkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenyl which may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
and
R² represents straight-chain or branched alkyl having 1 to 4 carbon atoms, where these radicals may be mono- to tetrasubstituted by identical or different substituents from the group consisting of fluorine, chlorine, bromine, methoxy, ethoxy, propoxy, isopropoxy, alkoximino having 1 or 2 carbon atoms in the alkoxy moiety, cyclopropyl, cyclobutyl, cyclopentyl and cyclohexyl,
or
represents straight-chain or branched alkenyl having 2 to 5 carbon atoms, where each of these radicals may be mono- to trisubstituted by identical or different substituents from the group consisting of fluorine, chlorine, bromine, methoxy, ethoxy, propoxy, isopropoxy, cyclopropyl, cyclobutyl, cyclopentyl and cyclohexyl,
or
represents cycloalkyl having 3 to 6 carbon atoms, where each of these radicals may be mono- to trisubstituted by identical or different substituents from the group consisting of fluorine, chlorine, bromine, cyano, methyl, ethyl, propyl, isopropyl and tert-butyl,
or
represents phenylalkyl having 1 to 4 carbon atoms in the straight-chain or branched alkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenyl which may be mono- to trisubstitued by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
**characterized in that**
a) in a first step, hydrazine derivatives of the formula 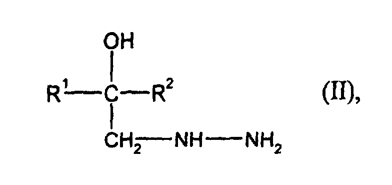 in which
R¹ and R² are each as defined above
are reacted with carbonyl compounds of the formula 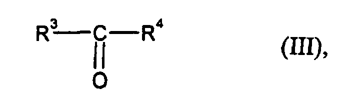 in which
R³ represents methyl, ethyl, n-propyl, isopropyl, tert-butyl or phenyl and
R⁴ represents hydrogen, methyl, ethyl or n-propyl or
R³ and R⁴ together represent a -(CH₂)₅-chain,
and with thiocyanate of the formula
X-SCN (IV),
in which
X represents sodium, potassium or ammonium,
if appropriate in the presence of a diluent and if appropriate in the presence of an acid, and
b) the resulting triazolidinethione derivatives of the formula 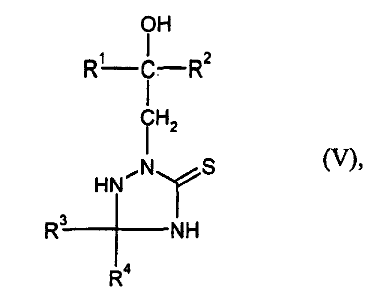 in which R¹, R², R³ and R⁴ are each as defined above
are reacted with formic acid, if appropriate in the presence of a catalyst and if appropriate in the presence of a diluent.

2. Process according to Claim 1, **characterized in that** the starting material used is 2-(1-chloro-cyclopropyl)-3-(2-chloro-phenyl)-2-hydroxy-propyl-1-hydrazine of the formula

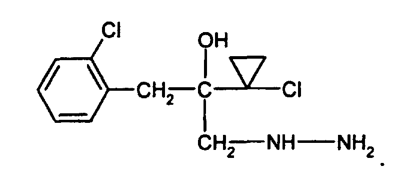

3. Process according to Claim 1, **characterized in that** the reaction component used for carrying out the first step is sodium thiocyanate or potassium thiocyanate.

4. Process according to Claim 1, **characterized in that** the first step is carried out at temperatures between 0°C and 120°C.

5. Process according to Claim 1, **characterized in that** the second step of the process according to the invention is carried out at temperatures between 80°C and 150°C.

6. Triazolidinethione derivatives of the formula 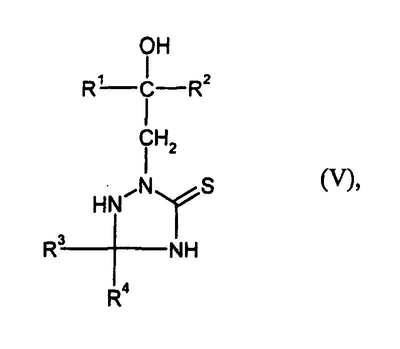 in which
R¹, R², R³ and R⁴ are each as defined in Claim 1.

7. Triazolidinethione derivative according to Claim 6, **characterized by** the formula

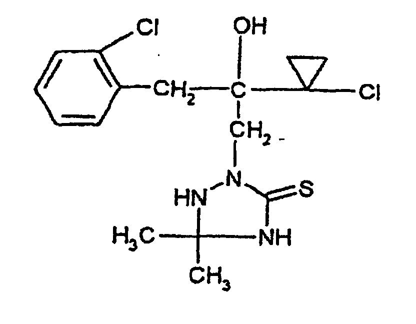

8. Process for preparing triazolinethione derivatives of the formula 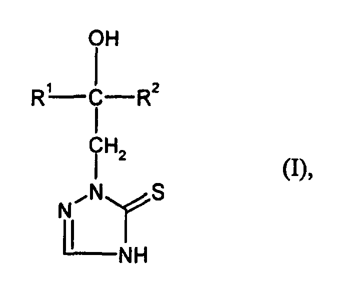 in which
R¹ and R² are each as defined in Claim 1,
**characterized in that**
- 1-chloro-2-hydroxy-ethane derivatives of the formula 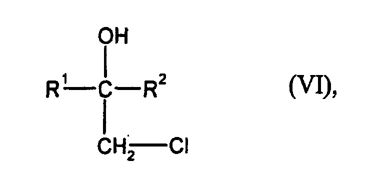 or oxirane derivatives of the formula 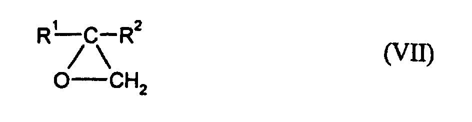 in which
R¹ and R² are each as defined above
are reacted with hydrazine hydrate, if appropriate in the presence of a diluent, and the resulting
- hydrazine derivatives of the formula 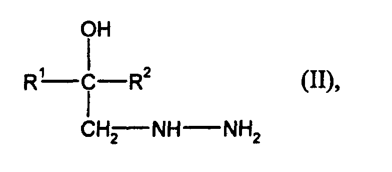 in which
R¹ and R² are each as defined above
are reacted without prior isolation with carbonyl compounds of the formula 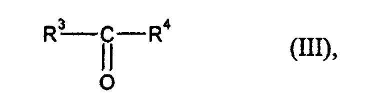 in which
R³ and R⁴ are each as defined in Claim 1,
and with thiocyanate of the formula
X-SCN (IV),
in which
X represents sodium, potassium or ammonium,
if appropriate in the presence of a diluent and if appropriate in the presence of an acid, and
- the resulting triazolidinethione derivatives of the formula 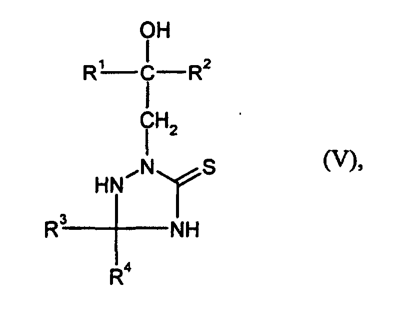 in which
R¹, R², R³ and R⁴ are each as defined above
are reacted with formic acid, if appropriate in the presence of a catalyst and if appropriate in the presence of a diluent.

## Revendications

1. Procédé de production de dérivés de triazolinethione de formule 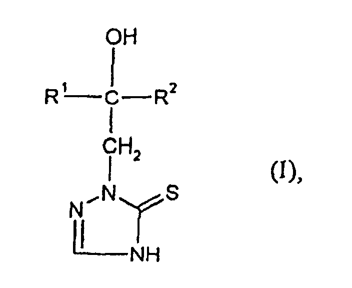 dans laquelle
R¹ est un reste alkyle linéaire ou ramifié ayant 1 à 4 atomes de carbone, ces restes pouvant porter 1 à 4 substituants, identiques ou différents, fluoro, chloro, bromo, méthoxy, éthoxy, propoxy, isopropoxy, alkoximino ayant 1 ou 2 atomes de carbone dans la partie alkoxy, cyclopropyle, cyclobutyle, cyclopentyle et/ou cyclohexyle,
ou bien
un reste alcényle linéaire ou ramifié ayant 2 à 5 atomes de carbone, chacun de ces restes pouvant porter 1 à 3 substituants, identiques ou différents, fluoro, chloro, bromo, méthoxy, éthoxy, propoxy isopropoxy, cyclopropyle, cyclobutyle, cyclopentyle et/ou cyclohexyle,
ou bien
un reste cycloalkyle ayant 3 à 6 atomes de carbone, chacun de ces restes pouvant porter 1 à 3 substituants, identiques ou différents, fluoro, chloro, bromo, cyano, méthyle, éthyle, propyle, isopropyle et/ou tertiobutyle,
ou bien
un reste phénylalkyle ayant 1 à 4 atomes de carbone dans la partie alkyle linéaire ou ramifiée, la partie phényle pouvant porter 1 à 3 substituants, identiques
ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien un reste phényle qui peut porter 1 à 3 substituants, identiques ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
et
R² est un reste alkyle linéaire ou ramifié ayant 1 à 4 atomes de carbone, ces restes pouvant porter 1 à 4 substituants, identiques ou différents, fluoro, chloro, bromo, méthoxy, éthoxy, propoxy, isopropoxy, alkoximino ayant 1 ou 2 atomes de carbone dans la partie alkoxy, cyclopropyle, cyclobutyle, cyclopentyle et/ou cyclohexyle,
ou bien un reste alcényle linéaire ou ramifié ayant 2 à 5 atomes de carbone, chacun de ces restes pouvant porter 1 à 3 substituants, identiques ou différents, fluoro, chloro, bromo, méthoxy, éthoxy, propoxy, isopropoxy, cyclopropyle, cyclobutyle, cyclopentyle et/ou cyclohexyle,
ou bien
un reste cycloalkyle ayant 3 à 6 atomes de carbone, chacun de ces restes pouvant porter 1 à 3 substituants, identiques ou différents, fluoro, chloro, bromo, cyano, méthyle, éthyle, propyle, isopropyle et/ou tertiobutyle,
ou bien
un reste phénylalkyle ayant 1 à 4 atomes de carbone dans la partie alkyle linéaire ou ramifiée, la partie phényle pouvant porter 1 à 3 substituants, identiques
ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, éthoximinométhyle, 1-méthoximinoéthyle, nitro et/ou cyano,
ou bien
un reste phényle qui peut porter 1 à 3 substituants, identiques ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinomémthyle, 1-méthoximinoéthyle, nitro et/ou cyano,
**caractérisé en ce que** :
a) on fait réagir dans une première étape des dérivés d'hydrazine de formule 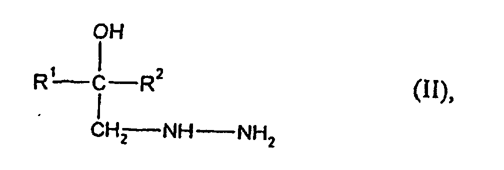 dans laquelle
R¹ et R² ont les définitions indiquées ci-dessus, avec des composés carbonyliques de formule 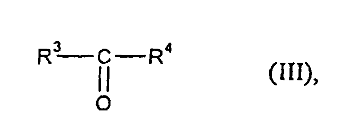 dans laquelle
R³ est un reste méthyle, éthyle, n-propyle, isopropyle, tertiobutyle ou phényle et
R⁴ est l'hydrogène, un reste méthyle, éthyle ou n-propyle, ou bien
R³ et R⁴ forment ensemble une chaîne -(CH₂)₅-,
et avec un thiocyanate de formule
X-SCN (IV)
dans laquelle
X représente le sodium, le potassium ou l'ion ammonium,
éventuellement en présence d'un diluant et en présence éventuelle d'un acide, et
b) on fait réagir les dérivés de triazolidinethione produits de formule 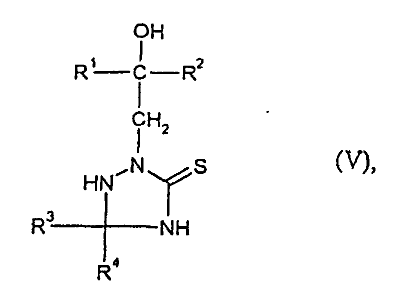
dans laquelle
R¹, R², R³ et R⁴ ont les définitions indiquées ci-dessus,
avec l'acide formique, éventuellement en présence d'un catalyseur et en présence éventuelle d'un diluant.

2. Procédé suivant la revendication 1, **caractérisé en ce qu'**on utilise comme composé de départ, la 2-(1-chlorocyclopropyl)-3-(2-chlorophényl)-2-hydroxypropyl-1-hydrazine de formule

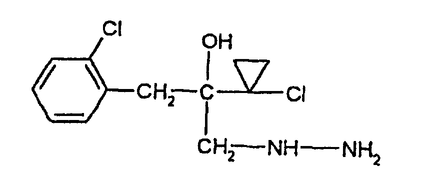

3. Procédé suivant la revendication 1, **caractérisé en ce qu'**on utilise comme composant réactionnel, dans la conduit de la première étape, le thiocyanate de sodium ou de potassium.

4. Procédé suivant la revendication 1, **caractérisé en ce qu'**on opère à des températures comprises entre 0°C et 120°C dans la conduite de la première étape.

5. Procédé suivant la revendication 1, **caractérisé en ce qu'**on opère à des températures comprises entre 80°C et 150°C dans la seconde étape du procédé de l'invention.

6. Dérivés de triazolidinethione de formule 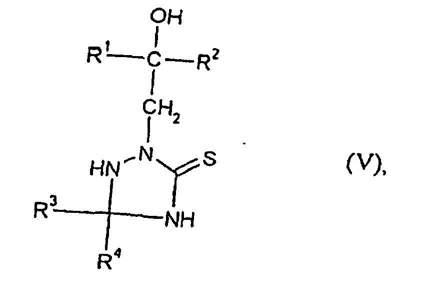 dans laquelle
R¹, R², R³ et R⁴ ont les définitions indiquées dans la revendication 1.

7. Dérivé de triazolidinethione suivant la revendication 6, **caractérisé par** la formule

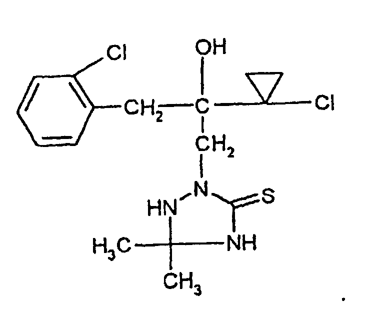

8. Procédé de production de dérivés de triazolinethione de formule 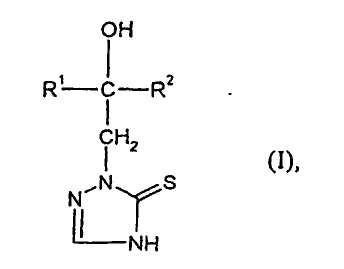 dans laquelle
R¹ et R² ont les définitions indiquées dans la revendication 1,
**caractérisé en ce qu'**on fait réagir
des dérivés de 1-chloro-2-hydroxyéthane de formule 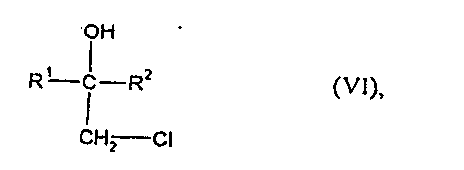 ou des dérivés d'oxiranne de formule 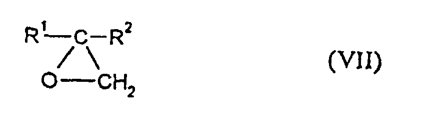 formules dans lesquelles
R¹ et R² ont les définitions indiquées ci-dessus, avec l'hydrate d'hydrazine, éventuellement en présence d'un diluant, et on fait réagir les dérivés d'hydrazine ainsi produits de formule 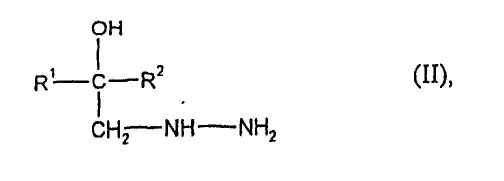 dans laquelle
R¹ et R² ont les définitions indiquées ci-dessus, sans isolement préalable, avec des composés carbonyliques de formule 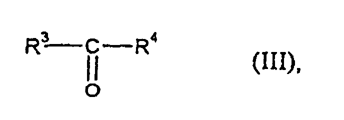 dans laquelle
R³ et R⁴ ont les définitions indiquées dans la revendication 1,
et avec un thiocyanate de formule
X-SCN (IV)
dans laquelle
X représente le sodium, le potassium ou l'ion ammonium,
éventuellement en présence d'un diluant et en présence éventuelle d'un acide, et
on fait réagir les dérivés de triazolidinethione produits de formule 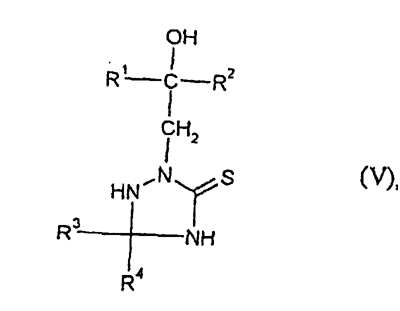 dans laquelle
R¹, R², R³ et R⁴ ont les définitions indiquées ci-dessus,
avec l'acide formique, éventuellement en présence d'un catalyseur et en présence éventuelle d'un diluant.
